# EUROPÄISCHE PATENTANMELDUNG

(11) **EP 0 686 644 A2**
(43) Veröffentlichungstag der Anmeldung: **13.12.1995**
(21) Anmeldenummer: 95108051.4
(22) Anmeldetag: 26.05.1995
(51) Int. Cl.: C08B 37/16, C07C 13/70

(54) **Verfahren zur Herstellung von Fulleren-Einschlussverbindungen**

(30) Priorität: 08.06.1994 DE 4419981
(71) Anmelder: HOECHST AKTIENGESELLSCHAFT, D-65929 Frankfurt am Main (DE)
(72) Erfinder: Braun, Tibor, Prof. Dr., HU-1144 Budapest (HU); Barcza, Lajos, Prof. Dr., HU-1112 Budapest (HU); Buvari-Barcza, Agnes, Prof. Dr., HU-1112 Budapest (HU)

(57) **Zusammenfassung**

Die Erfindung betrifft ein Verfahren zur Herstellung von einer Einschlußverbindung aus C₆₀-Fulleren und einem Wirtsgitter ausgewählt aus γ-Cyclodextrin und Calix-[8]-aren, das dadurch gekennzeichnet ist, daß die Einschlußverbindung durch mechanochemische Aktivierung eines Feststoffgemisches, enthaltend C₆₀-Fulleren und das Wirtsgitter, gebildet wird.

## Beschreibung

Zahlreiche Untersuchungen an Fullerenen wurden, bedingt durch deren hydrophoben Charakter in organischen Lösemitteln durchgeführt, obwohl die Fullerene in den meisten dieser Lösemittel nur schlecht löslich sind (N. Sivaraman et al., J. Org. Chem. 1992, 57, 6077-6079).

Kürzlich gelang es C₆₀-Fulleren auch in Wasser zu lösen, indem festes C₆₀-Fulleren und eine wäßrige γ-Cyclodextrin-Lösung auf Siedetemperatur erhitzt wurden und unter Rückfluß nach 50 Stunden und mehr in eine γ-Cyclodextrin-C₆₀-Fulleren-Einschlußverbindung überführt wurden (M. Sundahl et al., Synthetic Metals, 55-57, 1993, 3252-3257; T. Andersson et al., J. Chem. Soc., Chem. Commun. 1992, 604).

Wasserlösliche Calixaren-C₆₀-Fulleren-Einschlußverbindungen, hergestellt durch eine 32-stündige Reaktion von C₆₀-Pulver und einer wäßrigen Calixaren-Lösung unter Rückfluß, werden von R.U. Williams et al. (Recl. Trav. Chim. Pays-Bas 111, 531-532 (1992)) beschrieben.

Von Nachteil bei den beschriebenen Verfahren ist die langwierige Herstellung unter Rückflußbedingungen, die zu einer Schädigung des C₆₀-Moleküls führen kann.

Aufgabe ist es daher ein einfaches und schnelleres Verfahren zur Herstellung von wasserlöslichen Fulleren-Einschlußverbindungen zu entwickeln.

Gegenstand der Erfindung ist ein Verfahren zur Herstellung von einer Einschlußverbindung aus C₆₀-Fulleren und einem Wirtsgitter ausgewählt aus γ-Cyclodextrin und Calix-[8]-aren, dadurch gekennzeichnet, daß die Einschlußverbindung durch mechanochemische Aktivierung eines Feststoffgemisches, enthaltend C₆₀-Fulleren und das Wirtsgitter, gebildet wird.

Bei dem erfindungsgemäßen Verfahren wird festes C₆₀-Fulleren mit festem γ-Cyclodextrin bzw. festem Calix-[8]-aren mechanochemisch behandelt, d.h. innig vermischt beispielsweise durch mechanisches Mörsern oder Anwendung einer Kugelmühle. Das Molverhältnis von eingesetztem Fulleren zu γ-Cyclodextrin bzw. Calix-[8]-aren beträgt im allgemeinen 1:1 bis 1:15, bevorzugt 1:8 bis 1:12, besonders bevorzugt 1:10. Das erfindungsgemäße Verfahren wird im allgemeinen bei einer Temperatur von -20 bis 100°C, bevorzugt 20 bis 50°C, besonders bevorzugt 25°C, innerhalb von 0,5 bis 20 Stunden durchgeführt.

Das C₆₀-Fulleren kann in reiner Form (> 99 Gew.%), als Mischung (70 - 99 Gew. %) oder aber auch als Bestandteil von fullerenhaltigem Ruß (Anteil C₆₀ 1-15 Gew.%) eingesetzt werden. Das erfindungsgemäße Verfahren ist daher auch geeignet auf einfache Weise C₆₀-Fulleren von fullerenhaltigem Ruß abzutrennen.

Bevorzugt ist der Einsatz von reinem C₆₀-Fulleren.

Die Charakterisierung der Einschlußverbindung kann UV-spektroskopisch aus wäßriger Lösung erfolgen. Zum Zwecke der Charakterisierung kann im Anschluß an die mechanochemische Behandlung des Feststoffgemisches dieses durch Zugabe von 10 bis 30 ml deionisiertem Wasser in eine Suspension überführt werden. Diese Suspension wird im allgemeinen nochmals mechanochemisch behandelt beispielsweise durch Anwendung einer Kugelmühle (30 bis 80 Minuten) oder von Ultraschall (5 bis 10 Minuten). Während dieses Vorgangs geht die durch das erfindungsgemäße Verfahren gebildete Einschlußverbindung in Lösung.

Danach kann die die Einschlußverbindung enthaltende Lösung durch gängige physikalische Methoden (Zentrifugieren, Dekantieren u.ä.) abgetrennt und charakterisiert werden.

Die Einschlußverbindungen aus C₆₀-Fulleren und einem Wirtsgitter ausgewählt aus γ-Cyclodextrin und Calix-[8]-aren können beispielsweise als Photokatalysatoren verwendet werden.

Die verwendeten Wirtsgitter γ-Cyclodextrin und Calix-[8]-aren sind käuflich beispielsweise bei der Firma Chinon (Budapest) bzw. Sigma Chemical Co. (St. Louis, USA) erhältlich. C₆₀-Fulleren ist ebenfalls käuflich erhältlich (Firma Hoechst AG) oder wird nach dem von Krätschmer und Huffman bekannten Verfahren (Krätschmer W., Lamb L.D., Fostiropoulos K., Huffman D.R.: Nature 347 (1990) 354) aus fullerenhaltigem Ruß hergestellt.

### Beispiel 1

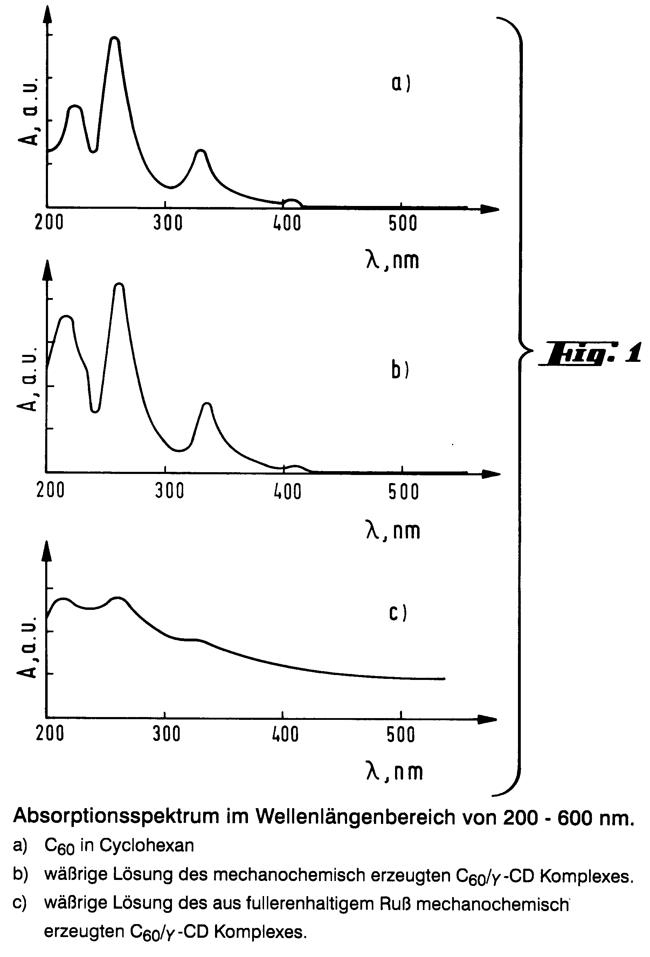

Eine homogene Mischung aus 10 mg C₆₀ (Hoechst AG) von über 99,4 %iger Reinheit und 100 mg γ-Cyclodextrin [γ-CD] (Chinoin, Budapest, Ungarn) wurde in einer Kugelmühle (Pulverisette Typ 701, Firma Fritsch, Deutschland) für 20 Stunden gemahlen. Anschließend wurde die Mischung mit 30 ml deionisiertem Wasser für eine weitere Stunde gemahlen. Die Suspension wurde anschließend zentrifugiert, der violett gefärbte Überstand wird mit Wasser auf 50 ml aufgefüllt, und das Absorptionsspektrum in einem UV/Vis Spektrometer (Perkin-Elmer Lambda 9) bestimmt (s. Abb. 1b).

### Beispiel 2

Eine homogene Mischung aus 30 mg fullerenhaltigem Ruß (hergestellt nach dem Verfahren von Krätschmer und Huffman) mit einem Gehalt an Fulleren von ca. 7 % und 300 mg γ-CD (Chinon, Budapest, Ungarn) wurde in einer Kugelmühle (Pulverisette, Typ 701, Firma Fritsch, Deutschland) für 10 Stunden gemahlen.

Anschließend wurde die Mischung mit 30 ml deionisiertem Wasser für eine weitere Stunde gemahlen. Die Suspension wurde zentrifugiert, der violett gefärbte Überstand wurde mit Wasser auf 50 ml aufgefüllt und das Absorptionsspektrum in einem UV/Vis Spektrometer (Perkin-Elmer Lambda 9) bestimmt (s. Abb. 1c).

## Patentansprüche

1. Verfahren zur Herstellung von einer Einschlußverbindung aus C₆₀-Fulleren und einem Wirtsgitter ausgewählt aus γ-Cyclodextrin und Calix-[8]-aren, dadurch gekennzeichnet, daß die Einschlußverbindung durch mechanochemische Aktivierung eines Feststoffgemisches, enthaltend C₆₀-Fulleren und das Wirtsgitter, gebildet wird.

2. Verfahren nach Anspruch 1, dadurch gekennzeichnet, daß das Molverhältnis von C₆₀-Fulleren und Wirtsgitter 1:1 bis 1:15 beträgt.

3. Verfahren nach Anspruch 1 oder 2, dadurch gekennzeichnet, daß als Wirtsgitter γ-Cyclodextrin eingesetzt wird.

4. Verwendung einer Einschlußverbindung aus C₆₀-Fulleren und einem Wirtsgitter ausgewählt aus γ-Cyclodextrin und Calix-[8]-aren als Photokatalysator.
